# EUROPEAN PATENT APPLICATION

(11) **EP 3 756 653 A1**
(43) Date of publication of application: **30.12.2020**
(21) Application number: 19183309.4
(22) Date of filing: 28.06.2019
(51) Int. Cl.: A61K 9/14, A61K 31/495

(54) **MICROPARTICULATE VARENICLINE CITRATE**

(71) Applicant: Alfred E. Tiefenbacher (GmbH & Co. KG), 22767 Hamburg (DE)
(72) Inventor: Ogibalov, Ivan, 50410 Tartu (EE); Uustare, Ain, 50410 Tartu (EE); Fitzner, Ansgar, 20253 Hamburg (DE)
(74) Representative: Hamm&Wittkopp Patentanwälte PartmbB

(57) **Abstract**

Microparticulate varenicline citrate hydrate (polymorph A), methods reliably yielding this new form, and pharmaceutical compositions comprising microparticulate varenicline citrate hydrate, which stand out due to their excellent content uniformity.

## Description

### Background

The present disclosure, with the invention as defined in the independent claims and in preferred form in the dependent claims, relates to a novel solid form of 7,8,9,10-tetrahydro-6,10-methano-6H-pyrazino[2,3-h]-[3] benzazepine citrate, in particular polymorph A.

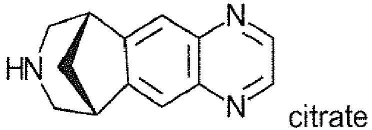

The compound, 7,8,9,10-tetrahydro-6,10-methano-6H-pyrazino[2,3-h]-[3] benzazepine citrate (hereinafter referred to as "varenicline citrate") is a high-affinity partial agonist of the *α*₄*β*₂ nicotinic acetylcholine receptor subtype (nACh) that leads to the release of dopamine in the *nucleus accumbens* when activated, and, therefore, varenicline has the capacity to reduce the feelings of craving and withdrawal caused by smoking cessation. Thus, varenicline and its pharmaceutically acceptable salts are used in the treatment of nicotine addiction.

In said treatment, varenicline is administered in dosages of 0.5-2 mg daily, whereby the drug is provided in dosage forms comprising as little as 0.5 or 1 mg varenicline (as free base). As it is the case for every active pharmaceutical ingredient (API) that is administered in such low dosages, achieving batch uniformity of the API itself, blend uniformity of the drug admixed with the excipients, and content uniformity of the API within the final dosage form represent common problems in the formulation of varenicline citrate. This problem is addressed by the present invention.

Batch uniformity summarizes the macroscopic properties of the drug after its preparation and includes the product's water content, crystal state, particle size distribution, specific surface area, aspect ratio etc. Batch uniformity allows that the product of each batch can be processed into the drug/excipient-blend and into the final dosage form without any batch-to-batch adaptation. Blend uniformity refers to the uniformity of the active pharmaceutical ingredient within the bulk-mixture of the drug with the excipients. Batch uniformity represents a mandatory precondition for content uniformity - in particular in the case of dosage forms with low drug load. Content uniformity ensures that a consistent dose of the API is maintained between single dosages so that the patient receives the correct and same dose each time the medicament is administered. It is commonly known that the particle size of the API has an impact on its batch uniformity, on the blend uniformity of its mixture with excipients and/or on the content uniformity of the final dosage form; concerning all these issues, smaller particle sizes are generally preferred.

Highly advantageous crystalline forms of varenicline citrate are described in European patent EP 1 390 366, which are denoted as hydrate form A and anhydrous form B. Both polymorphs show good aqueous solubility of > 100 mg/ml and a native pH of 3.7 in aqueous solution. However, upon micronization - a process step that is regularly applied and usually necessary to obtained small drug particles and to provide batch uniformity and to support blend as well as content uniformity - hydrate form A of varenicline citrate transforms into anhydrous form B (EP 1 390 366, example 2). The anhydrous form B may re-absorb some water upon formulation or storage of the final dosage form. Thus, micronization of form A or use of form B of varenicline citrate results in a product with non-stoichiometric water-content rendering the provision of an API with constant properties (batch uniformity), of a drug/excipient-admixture with the varenicline citrate being uniformly dispersed in the bulk composition (blend uniformity), and of a final dosage form comprising exactly the right amount of the active agent (content uniformity) very problematic. The more or less complete loss of crystal-water during micronization and the re-absorption of some water from the environment during formulation and storage of the dug/excipient-blend and of the final dosage form may result in a water-content varying from batch-to-batch, which, as a consequence, requires batch-to-batch adaptions when formulating the final dosage form to achieve the required content uniformity.

According to European patent EP 1 390 366, polymorph A of varenicline citrate is obtained by treating a solution of varenicline with a solution of citric acid (EP 1 390 366, example 1). Polymorph B may be obtained by reducing the particle size of the product of example 1 via micronization (EP 1 390 366, example 2).

Therefore, there was a need for the provision of a form of varenicline citrate allowing for yielding constant batch uniformity of the active ingredient and, thus, achieving a sufficient blend uniformity of the active ingredient within the excipient-mixture as well as a desired content uniformity of the active ingredient within the final dosage form.

### Summary of the invention

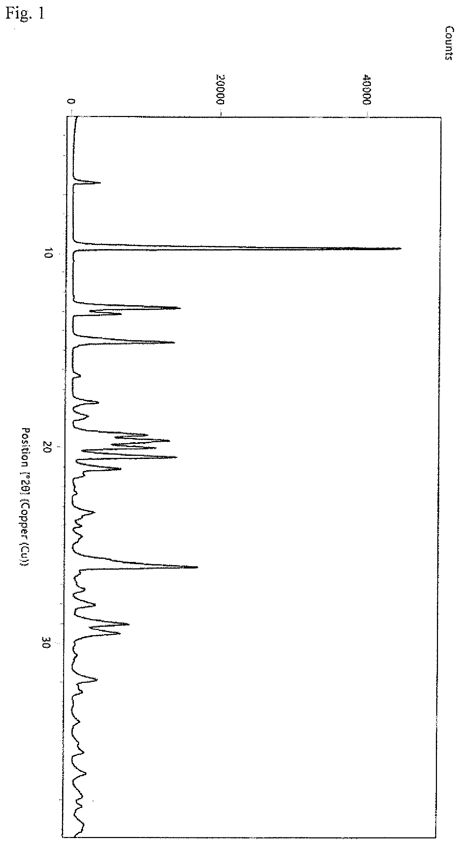

The present invention relates to a new form of the citrate salt of 7,8,9,10-tetrahydro-6,10-methano-6H-pyrazino[2,3-h]-[3] benzazepine in its polymorphic form A, which is characterized by powder X-ray diffractions (PXRD) peaks at 9.8°, 12.8°, 14.6°, 19.6°, 20.0°, 20.5°, and 26.1° ± 0.2° (2θ). Furthermore, the hydrate form A of varenicline citrate is characterized by its (PXRD) pattern depicted in fig. 1. Moreover, the present invention provides a method reliably yielding the new form of varenicline citrate and it encompasses pharmaceutical compositions that stand out due to their highly advantageous content uniformity and very consistent properties, such as their desirably constant water content.

### Detailed description of the invention

In their extensive research and analyses, the inventors have surprisingly found that when a solution of varenicline is added to a solution of citric acid, the citric acid salt of varenicline precipitates in microparticulate form rendering the micronization of the API fully dispensable. In a preferred embodiment a mixture of isopropanol and water is used for preparing the varenicline solution as well as for the citric acid solution, in a more preferred embodiment isopropanol and water are used in a ratio of 50:50 to 80:20, in a most preferred embodiment an isopropanol/water-mixture with a ratio of 80:20 is used for the varenicline solution as well as for the citric acid solution. Preferably the varenicline solution is added to the citric acid solution under vigorous stirring and/or at room temperature. In a preferred embodiment 3.7-6.4 ml solvent mixture is used per gram of varenicline as well as of citric acid, respectively. Most preferably about 5 ml solvent mixture is used per gram of varenicline and citric acid.

After drying at increased temperature and/or reduced pressure, varenicline citrate is obtained in form of a pale yellow precipitate. Preferably, the precipitate is dried in a vacuum oven at a temperature of 40-50 °C and/or at a pressure of 5-150 Torr, preferably below 5 Torr.

The obtained pale yellow precipitate is obtained in microparticulate form with a Dv(90)-value of below 100 µm, a Dv(50)-value of below 50 µm, and a Dv(10)-value of below 20 µm; preferably, a Dv(90)-value of below 60 µm, a Dv(50)-value of below 25 µm, and a Dv(10)-value of below 7 µm; and more preferred, the Dv(90)-value is below 45 µm, the Dv(50)-value is below 20 µm, and the Dv(10)-value is below 5 µm. The dried microparticulate product consistently has a water-content in the range of 2.0 % to 4,0 %, preferably between 3.0 % and 3,8 %.

The claimed microparticulate form A of varenicline citrate is characterized by PXRD peaks at 9.8°, 12.8°, 14.6°, 19.6°, 20.0°, 20.5°, and 26.1° ± 0.2° (2θ). It may be further characterized by additional PXRD peaks at 13.2°, 14.4°, 19.4°, 19.8°, 21.1°, 25.7°, 29.0°, and 29.5° ± 0.2° (2θ). The diffraction peaks at diffraction angles (2θ) in a measured powder X-ray diffraction analysis for the claimed microparticulate form A are shown in Table I.

**Table I**

| **angle (2θ)** | **intensity** |
|---|---|
| 6.42 | 8.75 |
| 9.79 | 100.00 |
| 12.84 | 34.48 |
| 13.15 | 14.88 |
| 14.40 | 11.57 |
| 14.60 | 33.23 |
| 17.70 | 8.63 |
| 19.39 | 24.26 |
| 19.64 | 32.88 |
| 19.77 | 22.18 |
| 20.04 | 24.97 |
| 20.52 | 32.56 |
| 21.09 | 15.70 |
| 23.30 | 7.32 |
| 25.69 | 11.98 |
| 26.10 | 40.37 |
| 29.04 | 18.83 |
| 29.53 | 15.27 |
| 31.92 | 7.88 |

The claimed microparticulate varenicline citrate is achieved as polymorphic form A in pure form and shows constant particle size distribution and constant water content. Thus, the product of the present invention shows formidable batch uniformity in the finished dosage form like a tablet, the API can be formulated into a bulk drug/excipient-mixture with a highly beneficial blend uniformity, and said drug/excipient-blend can be formulated into final dosage forms with impressive content uniformity - without the need for any batch-to-batch adaption of the final dosage form or of the process for its preparation. These highly desirable properties are achieved by a rather simple and straight forward approach and allow for avoiding time consuming and complicated techniques and methods, such as micronization, controlled re-hydration, or dry- or wet-granulations of the active pharmaceutical ingredient.

Finally, the highly advantages and consistent properties of the claimed form of varenicline citrate that are made available by the manufacturing process of the present invention are carried on to the pharmaceutical compositions that comprise the new product and that are made available by this invention. The pharmaceutical compositions comprising the new form of varenicline citrate are characterized by an excellent content uniformity and very consistent and predictable overall properties; for example, the formulations also have a very uniform water content.

### Synthesis of microparticulate varenicline citrate - form A

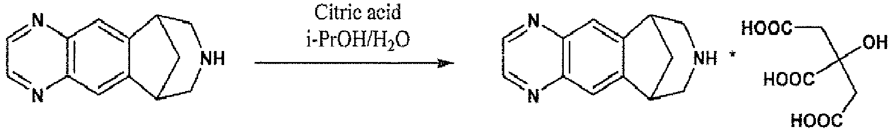

545.8 g (2.556 mol) of varenicline were dissolved in 2725 mL of an isopropanol/water mixture (80:20) at room temperature in a 5 L glass beaker; magnetic stirring was used. 554 g (2.811 mol, 1.1 eq.) of citric acid were dissolved in 2725 mL of an isopropanol/water mixture (80:20) at room temperature in a 10 L three-necked round-bottom flask equipped with mechanical stirrer and 2 L dropping funnel (for addition of the varenicline-solution). Over 30 min, the varenicline solution was added to the citric acid solution under vigorous stirring at room temperature. Spontaneous precipitation of varenicline citrate began almost immediately after the addition of the varenicline solution was initiated. The reaction mixture was stirred at room temperature for 3.5 hours.

The obtained solid of varenicline citrate was collected by filtration; the precipitate was washed thoroughly with isopropanol/water (80:20). The pale yellow precipitate was dried under vacuum (at a temperature of 45°C and a pressure of 50-150 Torr) for about 2 days. After sieving - breaking up agglomerated particles - a product with a weight of 1001.4 g was obtained (Varenicline Citrate, 96.1 % from theory; HPLC purity: 99.6 %).

### Powder X-ray diffraction analysis

The powder X-ray diffraction (PXRD) pattern for the obtained microparticulate varenicline citrate was collected using a PANalytical/Empyrean-diffractometer equipped with copper radiation CuK*_{α}* (1.5406 Å), at a generator settings of 45 kV and 40mA. Data was collected from 3.0 to 40.0 ° in 2θ using a step size of 0.013° and a step time of 73.695 s. During the measurement the sample was rotated at a speed 2s/revolution to improve counting statistics
The diffraction peaks at diffraction angles (2θ / 2Theta) in the measured powder X-ray diffraction analysis for the obtained microparticulate varenicline citrate are shown in Table II. It corresponds to the diffraction pattern of hydrate form A in EP 1 390 366. The relative intensities, however, may change depending on the crystal size and the morphology. The actual measured powder diffractogram is displayed in figure 1.

**Table II**

| **angle (2θ)** | **d-value (Å)** | **intensity** |
|---|---|---|
| 6.42 | 13.77 | 8.75 |
| 9.79 | 10.08 | 100.00 |
| 12.84 | 6.90 | 34.48 |
| 13.15 | 6.73 | 14.88 |
| 14.40 | 6.15 | 11.57 |
| 14.60 | 6.07 | 33.23 |
| 17.70 | 5.01 | 8.63 |
| 19.39 | 4.58 | 24.26 |
| 19.64 | 4.52 | 32.88 |
| 19.77 | 4.49 | 22.18 |
| 20.04 | 4.43 | 24.97 |
| 20.52 | 4.33 | 32.56 |
| 21.09 | 4.21 | 15.70 |
| 23.30 | 3.82 | 7.32 |
| 25.69 | 3.47 | 11.98 |
| 26.10 | 3.41 | 40.37 |
| 29.04 | 3.08 | 18.83 |
| 29.53 | 3.03 | 15.27 |
| 31.92 | 2.80 | 7.88 |

### Particle size distribution

Particle size distribution of the obtained varenicline citrate was analysed by laser diffraction with the following method: Equipment: Mastersizer 3000 + hydro Wet dispersion unit (Model MAZ3000 + Hydro MV); Lens: Reverse Fourier (convergent beam); Detector lens: Glass; Effective focal length: 300 mm; Particle type: Non-spherical; Dispersant: Isopar G, RI 1.420; Material: Varenicline citrate, RI 1.55; and Analysis: Mie and Fraunhofer scattering.

Procedure: approx. 1.5 g of soybean lecithin was dissolved in 200 ml of Isopar G at room temperature to obtain a lecithin solution. Approx. 30 mg of the varenicline citrate sample was mixed with 20 ml of lecithin solution and sonicated for 5 min. The sample was delivered drop by drop into measuring cell until obscuration 13-18 % and measurement was started to obtain the particle size distribution.

By default the Mastersizer measurement is fundamentally a measurement of the volume distribution - transforming the result into number distribution is a mathematical process. The measurement results were reported as Dv(10)-, Dv(50)-and Dv(90)-values, which are standard percentiles. Dv(50) is the size in micrometers (µm) at which 50% of the particles of the sample is smaller and 50% is larger. This value is also known the Mass Median Diameter (MMD) or the median of the volume distribution. The v in the expression Dv(50) shows that this refers to the volume distribution. Dv(10) corresponds to the size of particle below which 10% of the sample lies and Dv(90) to the size of particle below which 90% of the sample lies.

The obtained results of the produced varenicline citrate sample were as follows:
Dv(10): 3.44 µm;
Dv(50): 16.1 µm;
Dv(90): 30.2 µm.

### Water content

The water content of the obtained varenicline citrate was determined as 2.0% by volumetric Karl Fischer titration according to chapter 2.5.12 of European Pharmacopeia (Ph. Eur. 2.5.12).

### Content uniformity of two tablet batches manufactured with microparticulate varenicline citrate

| **S. No.** | **Ingredients** | **mg / tablet** | |
|---|---|---|---|
| | | **0.5 mg** | **1 mg** |
| | **Stage-A: Blending and Compaction** | | |
| 1 | Varenicline Citrate | 0.95 | 1.91 |
| 2 | Cellulose, Microcrystalline | 58.95 | 117.89 |
| 3 | Starch, Pregelatinised | 40.00 | 80.00 |
| 4 | Magnesium Stearate | 0.05 | 0.10 |
| | **Stage-B: Lubrication and Compression** | | |
| 1 | Magnesium Stearate | 0.05 | 0.10 |
| | **Core tablet weight** | **100.00** | **200.00** |
| | **Stage-C: Coating** | | |
| 1 | Opadry® White Film coating | 5.00 | - |
| 2 | Opadry® Blue Film coating | - | 10.00 |
| 3 | Water, Purified | q.s. | q. s. |
| **Coated tablet weight** | | **105.00** | **210.00** |

### Manufacturing process:

All the materials of stage-A are sifted through a suitable sieve and mixed in a blender for sufficient time. Above blend is compacted and the compacts are subjected to milling to generate granules. The milled granules are lubricated with the stage-B materials and compressed into tablets. Finally the tablets are coated with the film coating material of stage-C.

The 1 mg tablet batch was manufactured with microparticulate varenicline citrate having a Dv(10)-value of 3.19 µm, a Dv(50)-value of 15.3 µm, and a Dv(90)-value of 37.9 µm. The 0.5 mg tablet batch was manufactured with a mixture of two batches of microparticulate varenicline citrate having a Dv(10)-value of 3,19 µm, a Dv(50)-value of 15.3 µm, and a Dv(90)-value of 37.9 µm; and a Dv(10)-value of 2.82 µm, a Dv(50)-value of 15.8 µm, and a Dv(90)-value of 42.8 µm, respectively.

Both tablet batches showed excellent content uniformity as determined according to the method "content uniformity" described in chapter 2.9.40 of the European Pharmacopoeia (Ph. Eur.), as shown in Table III.

**Table III**

| **Uniformity of Content of Tablets** | | |
|---|---|---|
| **Tablet No.** | **0.5 mg strength** | **1 mg strength** |
| | **% Label claim** | |
| 1 | 105.4 | 95.5 |
| 2 | 94.4 | 104.9 |
| 3 | 105.0 | 101.0 |
| 4 | 98.2 | 99.8 |
| 5 | 100.5 | 98.0 |
| 6 | 96.8 | 95.7 |
| 7 | 106.0 | 101.5 |
| 8 | 100.8 | 98.1 |
| 9 | 99.7 | 100.4 |
| 10 | 104.4 | 103.1 |
| **Mean** | **101.1** | **99.8** |
| Min | 94.4 | 95.5 |
| Max | 106.0 | 104.9 |
| SD | 3.98 | 3.04 |
| % RSD | 3.9 | 3.0 |
| **Acceptance value** | **9.55** | **7.3** |
| **Tablet Assay (%)** | **102.6** | **101.6** |

## Claims

1. Microparticulate varenicline citrate having an XRPD-diffraction pattern with peaks at 9.8°, 12.8°, 14.6°, 19.6°, 20.0°, 20.5° and 26.1° ± 0.2° (2θ), and **characterized by** a Dv(50)-value of below 50 µm.

2. Microparticulate varenicline citrate according to claim 1 having a Dv(10)-value of less than 20 µm and/or a Dv(90)-value of less than 100 µm.

3. Microparticulate varenicline citrate according to claim 1 having a Dv(50)-value of below 25 µm.

4. Microparticulate varenicline citrate according to claim 1 or claim 3 having a Dv(10)-value of less than 7 µm and/or a Dv(90)-value of less than 60 µm.

5. Microparticulate varenicline citrate according to claims 1-2 in polymorphic form A.

6. Microparticulate varenicline citrate according to claims 1-3 with an XRPD pattern as depicted in fig. 1.

7. Microparticulate varenicline citrate according to claims 1-4 for reducing the feelings of craving and withdrawal caused by smoking cessation.

8. Method for the preparation of the microparticulate varenicline citrate according to claims 1-5 comprising the addition of a solution of varenicline to a solution of citric acid.

9. Method for the preparation of microparticulate varenicline citrate according to claim 6, wherein the solvent used in the preparation of the varenicline solution and/or of the citric acid solution comprises isopropanol and water.

10. Method for the preparation of microparticulate varenicline citrate according to claim 7, wherein the isopropanol and water are used in a ratio of 50:50 - 80:20; preferably, isopropanol and water are used in a ratio of 80:20.

11. Method for the preparation of microparticulate varenicline citrate according to claim 6 comprising drying the obtained precipitate at a temperature of 40° - 50° C and/or under reduced pressure of less than 150 Torr.

12. Pharmaceutical composition comprising microparticulate varenicline citrate in accordance with claims 1-5.
